Europäisches Patentamt

European Patent Office

Office européen des brevets

(11) Publication number: **0 162 573**
**A2**

(12) # EUROPEAN PATENT APPLICATION

(21) Application number: **85302667.2**

(22) Date of filing: **16.04.85**

(51) Int. Cl.⁴: **A 61 F 2/16**

(30) Priority: **17.04.84 US 601380**

(43) Date of publication of application:
**27.11.85 Bulletin 85/48**

(84) Designated Contracting States:
**AT BE CH DE FR GB IT LI NL SE**

(71) Applicant: **Hecht, Sanford D.**
**87 Levbert Road**
**Newton Center Massachusetts 02159(US)**

(72) Inventor: **Hecht, Sanford D.**
**87 Levbert Road**
**Newton Center Massachusetts 02159(US)**

(74) Representative: **Brunner, Michael John et al,**
**GILL JENNINGS & EVERY 53-64 Chancery Lane**
**London WC2A 1HN(GB)**

(54) **Eye implant.**

(57) An artificial lens (11) is cantilevered from a support rod (13) in the vitreous chamber of the eye with the other end of the support rod affixed to an anterior chamber lens (15), the support (14) for it, the sclera or (natural) lens capsule. There may be a number of support rods and a number of lenses cantilevered from support rods in the vitreous cavity.

Fig. 1

Sanford D. Hecht.        - 1 -        Ref: 90/2426/02

## EYE IMPLANT

The present invention relates in general to improving and/or maintaining vision and more particularly concerns novel apparatus and techniques for improving vision with eye implants. The invention may be used with or without other natural or artificial lenses and arranged for adjustment to achieve particular optical effects.

An example of a prior art aphakic intraocular implant is disclosed in an article entitled "Pars Plana Phacoprosthesis (aphakic intraocular implant): A Preliminary Report by Louis J. Girard on page 19 of OPTHALMIC SURGERY for January 1981. Among other prior art are U. S. Patent Nos. 2,834,023, 3,961,379, 3,992,563, 4,021,382, 4,028,082, 4,110,848, 4,122,556, 4,159,546, 4,205,518, 4,214,585, 4,242,760, 4,338,687, 4,340,979, 4,343,050 and 4,342,123.

It is an important object of this invention to provide methods and means for maintaining and/or improving vision with eye implants.

The purpose of these new eye implants according to the invention is to improve and/or maintain human (and animal) vision. There are three major categories of implants (implant systems):

1. Optical implants.

2. Chemical or drug delivery implants.

3. Implants to alter the intraocular pressure, as in glaucoma or ocular hypertension, and/or enable the removal of intraocular substances.

The implants may be combinations of these three as well, and may be implanted with the natural lens and/or vitreous in place or wholly or in part removed, and different categories may be placed at the same or different surgeries. Secondary implanting (implanting after previously healed or partly healed eye surgery) may be done after any previous eye surgery.

The intraocular implants are implants which wholly or partly are implanted within the globe and/or its cornea and/or sclera or combination of these. The globe is the cornea, sclera and its contained parts. Such implants may be wholly or partly in the anterior chamber, posterior chamber, vitreous cavity or combinations of these.

Extraocular implants are implants placed episclerally, subconjunctivally, subTenon's capsule, orbitally, periorbitally, or combinations of these. Multiple implants may be placed intraocularly, episclerally, subconjunctivally, subTenon's capsule, orbitally or periorbitally to improve or maintain a given eye's vision.

Although the three major categories of implants listed earlier are generally intraocular (within the eye or globe), extraocular implants physically separated from intraocular or other extraocular implants may also be implanted which function alone or aide the action of the intraocular implants or other extraocular implants. Extraocular and intraocular implants may have physical connection with each other and/or affect each other via magnetic fields, electromagnetic radiation and ultrasound waves (vibrations) or combinations of these. All or some of these implants may be affected by other units, separate or attached, to the patient.

The invention will be better understood with reference to certain terms defined as follows:

An OPTIC is any part which alters the direction and/or amplitude of light (including ultraviolet and infrared radiation). Examples of optics are filters, mirrors, lenses, prisms, and occluders. Some optics may have multiple optical characteristics (e.g. a lens which polarizes light). Wavelengths of light and other electromagnetic radiation may be filtered by coatings of optics and also additives to their substance or by the substance used to make the optic itself.

OPTICS may be made wholly or partly of glass or plastic or comparable substances of various densities and refractive indexes. Other substances which enable similar or identical function may be used.

OPTICS may be made in many shapes and sizes and powers, and more than one type and combinations of the types may be present in an eye. All this is unique.

GAS or LIQUID or VACUUM OPTICS may be part of the optics or optical systems. Such gases or liquids may buoy the optics or implants (make them lighter) and by buoyancy aid in the movement of optics and/or other parts. They enable special effects such as increasing the refraction of light. They may interact with non-gaseous or nonliquid type optical implants or optical implant parts.

HAPTICS are those parts adjacent to and supporting the optic. The haptics may connect the optic to feet, arms or cantilevers.

DEFORMABLE and ELASTIC PARTS -- any part of these implants may have deformability or have elastic properties, or both. Some parts such as optics may be both deformable and elastic so as to change shape and return to their original shape (be reformable or have "a memory") when acted on or not acted on by other implant parts and thereby alter their optical properties such as their power. Such optics may be made wholly or partly of silicone rubber (plastic).

Parts may be malleable enabling preoperative, intraoperative and postoperative part modification.

SUPPORT STRUCTURES, OPTICS, AND THEIR MATERIALS -- such as feet, bearings, sleeves, stops partial frictional stops, catches, slides, levers, cantilevers, double wall through and through supports, haptics, hinges, ratchets & pinions, extensions, pins, pivots, arms guides, hydraulic systems, valves, screws, gears, springs, cams, pistons, etc., of implants of all types may be made of glass, silicone rubber, of Teflon® or other plastic, metal, or substances capable of equivalent or similar function and may be combinations of these and may have varied form and structure and weights and sizes, and may be flexible, deformable, elastic or not. Parts may have special surface modifications (coatings or machining) to alter light reflections. Some implants may be made entirely of one substance.

Numerous other features, objects and advantages of the invention will become apparent from the following specification when read in connection with the accompanying drawing in which:

FIGS. 1-5 show diagrammatic sectional views of vitreous cavity lenses supported from the anterior chamber lenses or haptics;

FIG. 6 is a diagrammatic sectional view of a separate vitreous cavity sclera supported lens implant;

FIGS. 7 and 9 are diagrammatic sectional views of vitreous cavity lenses supported from the sclera;

FIG. 8 shows a vitreous cavity lens implant supported by a posterior chamber capsule fixed lens implant;

FIGS. 10 and 14 show front views of anterior chamber lenses with fixed and moveable optics which may be placed in the right and left eyes, respectively;

FIG. 11 is a diagrammatic sectional view of vitreous cavity lenses supported by an angle supported anterior chamber lens;

FIGS. 12A and 12B are side and front views respectively of a solid element between hollow elements;

FIGS. 13A and 13B are side and front views, respectively, of a hollow element between solid elements;

FIG. 15 is a front view of a sliding optic;

FIG. 16 shows a ridged support rod;

FIGS. 17A and 17B are side and front views, respectively, of an anterior chamber supported vitreous cavity optic;

FIG. 18 shows a relationship between an extraocular and intraocular implants;

FIGS. 19A and 19B show front and side views, respectively, of a shape-changing variable focus optical implant;

FIG. 20 shows a rotatable disk implant with three optics;

FIG. 21 shows a depot on a cantilever leg in the vitreous cavity;

FIG. 22 shows a sclerally fixed type implant which has stabilizing foot extensions;

FIG. 23 shows a transverse double wall support with intrascleral feet;

FIG. 24 illustrates a foot entering a pocket in the sclera,

FIG. 25 shows an extensible lens support;

FIGS. 26A and 26B show plan and side views, respectively, of the cantilevered lens assembly for attachment in the pocket as shown in FIG. 24; and

FIGS. 27-32 show steps in an implant procedure according to the invention.

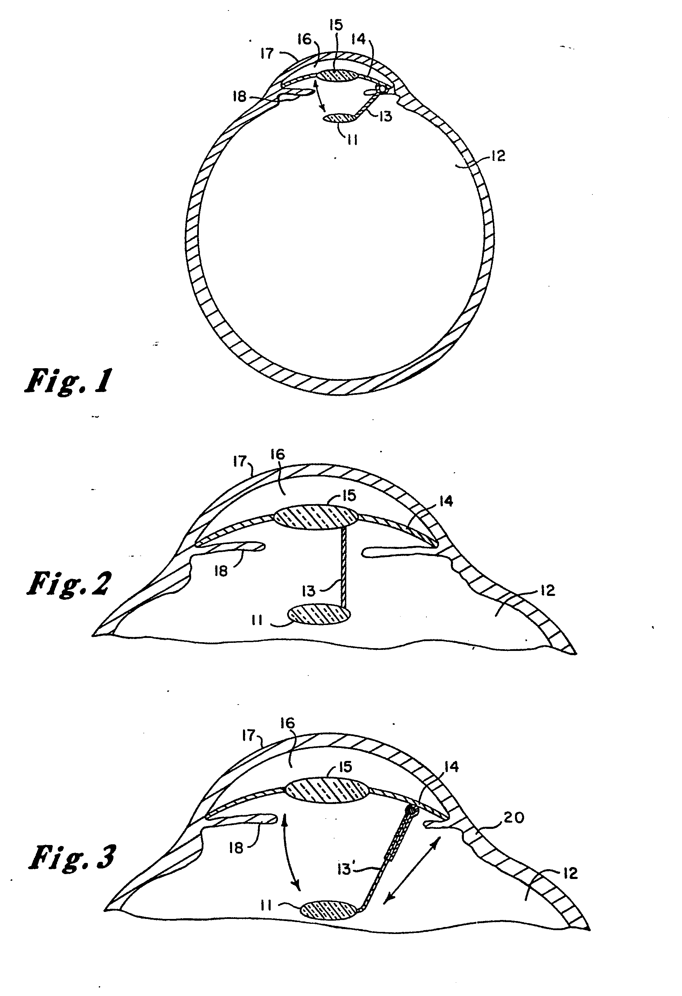

With reference now to the drawing and more particularly FIG. 1 thereof, there is shown a diagrammatic sectional view of the eye with a hinged vitreous cavity lens 11 in vitreous chamber 12 cantilevered from support rod 13 attached to haptic 14 of anterior chamber lens 15 in the anterior chamber 16 between the cornea 17 and iris 18. The hinged lens may move in or out of the visual axis.

Referring to FIG. 2, there is shown an alternate embodiment of the invention in which support rod 13 is suspended from the optic of anterior chamber lens 15.

Referring to FIG. 3, there is shown another embodiment of the invention with telescoping support rod 13' attached to the haptic 14 of an angle supported anterior chamber lens 15.

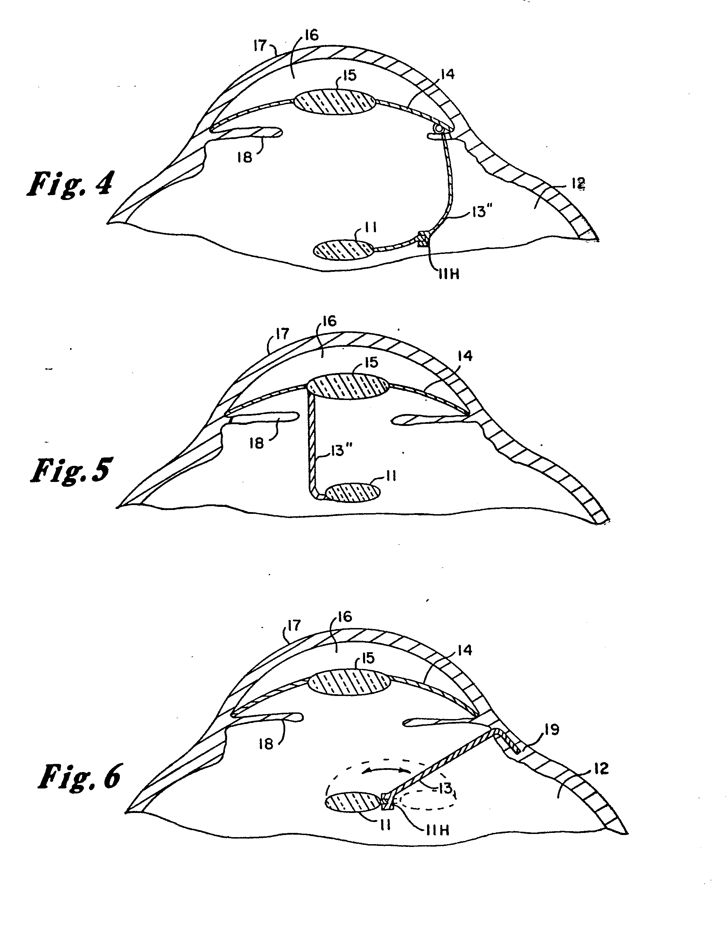

Referring to FIG. 4, there is shown still another embodiment of the invention with vitreous cavity lens 11 supported from haptic 14 by a curved support rod 13' to lessen the shadow on the retina and having lockable hinges to ease implant insertion.

FIG. 5 shows another embodiment of the invention with support rod 13" attached to an angle supported anterior chamber lens.

Referring to FIG. 6, there is shown another embodiment of the invention in which support rod 13 is fixed to sclera 19 by an intrascleral foot, and hinged optic 11 may move in and out of the visual axis.

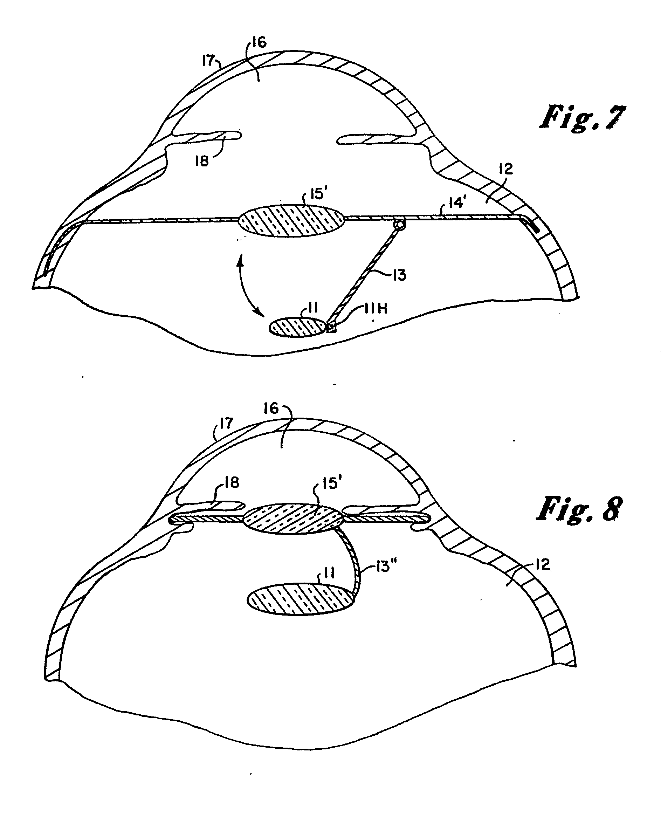

Referring to FIG. 7, there is shown another embodiment of the invention with support rod 13 attached to haptic 14' of vitreous cavity lens 15'.

Referring to FIG. 8 there is shown another embodiment of the invention with curved support rod 13" attached to a posterior chamber lens 15'.

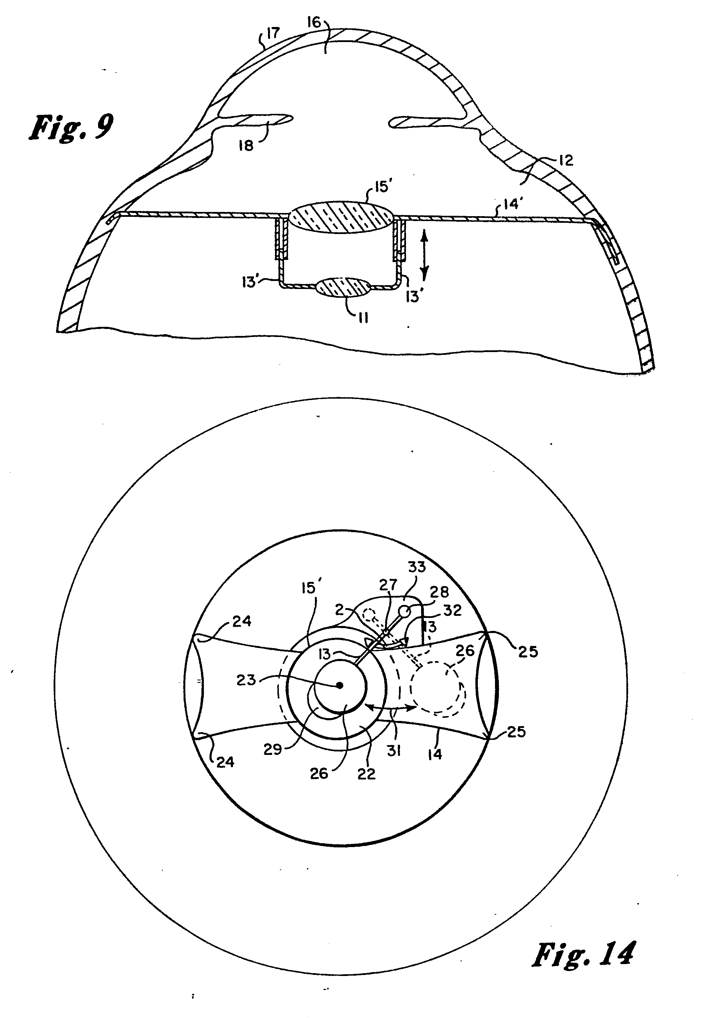

Referring to FIG. 9, there is shown another embodiment of the invention with two support rods 13' supporting vitreous chamber lens 11 from vitreous chamber lens 15'. Rods 13' may comprise permanent magnets which may be acted upon by external magnets to cause A-P (anterior-posterior) movement.

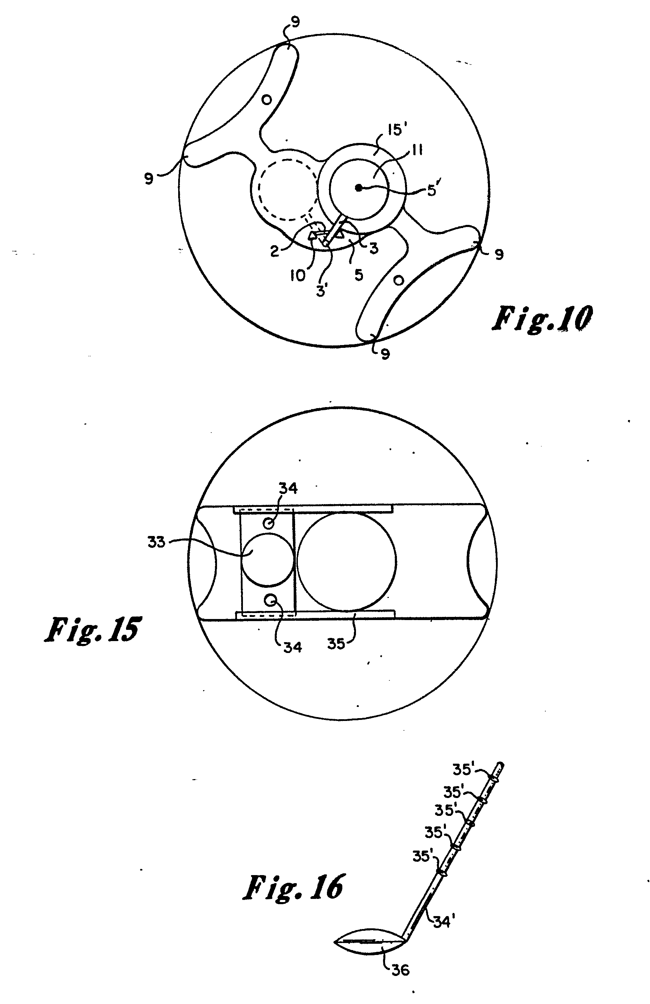

FIG. 10 shows a front view (anterior-posterior) of an anterior chamber implant with optics decentered into the visual A-P 5', held in place by its feet 9 in the angle and having a nonmoveable optic 15' and a moveable optic 11 connected to a hinged arm 3 which is fixed to a haptic 5 and which has a guide 2 and has stops 10 to limit its movement.

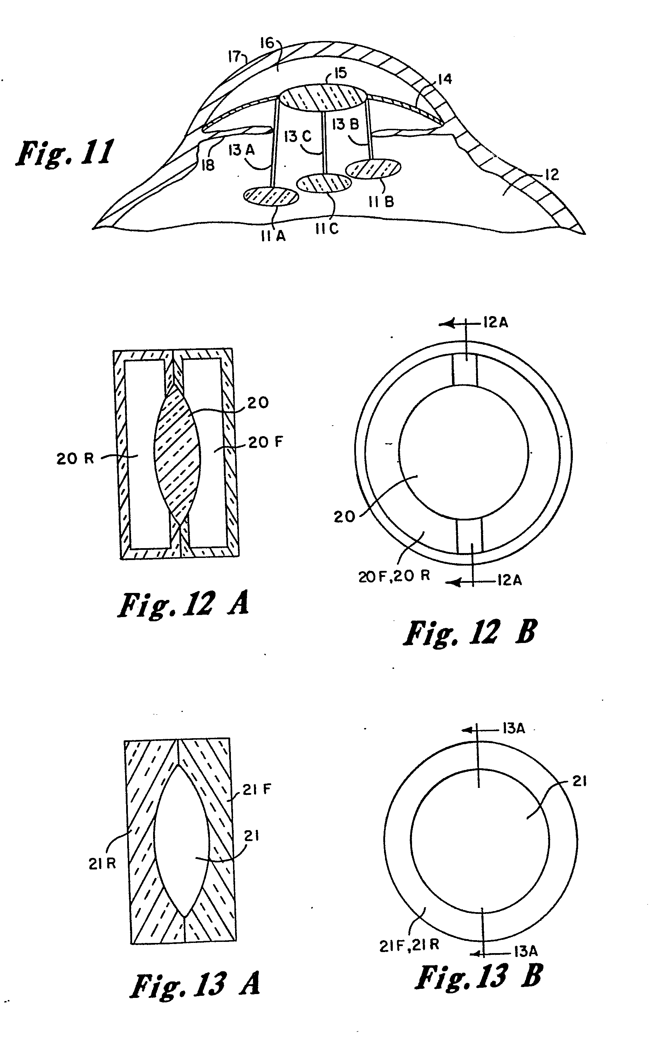

Referring to FIG. 11, there is shown a diagrammatic sectional view of another embodiment of the invention with three vitreous chamber lenses 11A, 11B and 11C cantilevered from rods 13A, 13B and 13C, respectively. Support rod 13A and 13B are attached to the haptic 14, and support rod 13C is attached to the optic of anterior chamber lens 15.

Some implants may require limbal incisions as large as 350°, while others may be inserted through more traditional size incisions.

The invention involves a number of novel concepts. There are cantilevered supports which may be fixed to the sclera intra-, epi-sclerally or endosclerally and wholly or partly fixed intra-corneally with a foot or feet, and multiple moveable cantilevers with or without lockable hinges or moveable parts which may put optic or optics into or alongside the visual axis. There may be multiple artificial implant optics in a given eye. The optics may be plus or minus power, prisms, graded density lenses, Fresnel lenses, aspheric lenses, tintedlenses and optics to correct human astigmatism, inter alia. The implants in the vitreous cavity may be off the visual axis of the eye, even if the natural lens has been removed. The vitreous cavity optics may be fixed in position by connection to anterior or posterior chamber lenses, or the sclera or cornea.

A vitreous chamber lens or other optics may coact with at least one other lens or optic to produce various effects, such as a telescopic magnification effect for near and also for distance and also minification of images in addition to the use of converging lenses or other optics for magnification. They may coact with external (such as eyeglasses) optical devices to produce standard and unusual optical effects, such as forming a telescope.

As with other implants, the vitreous cavity lenses or other optics or parts may be later movable by means such as movable cantilevered part or parts with and without projections or variations along their surfaces, or bends in the cantilever for engagement of instruments to change the position of optics or parts in the eye after installation. Optics or parts may also be moved by head or eye movements (patient's etc. volition) as described below. The vitreous cavity implants may be cantilevered off other cantilevers or fixated cross-bars in the eye, or anterior or posterior chamber implants.

The vitreous cavity anterior and posterior chamber implants may be inserted into the eye and sclerally supported more posteriorly through a pre-sealed retina and/or ablated retina and/or choroid for more stability with some implants. These implants may be placed with vitreous removed or not removed and may be located there with the natural eye lens remaining in place. The foot of the support rod may be globe-fixed at adjacent to, or at a distance from the entry incision forthe implant.

Typical prior art intraocular lenses have been placed in the same position as the natural lens or in front of it as fixed converging lenses to act like the natural lens which had been removed or was being simultaneously removed. Lenses according to the invention may be minus lenses and may be placed in multiple combinations to achieve certain effects, such as telescope effects. Lenses according to the invention may be movable both by the patient's and surgeon's actions, where the latter intrudes into the eye to move the lens to new location to achieve improved or new functions. With prior art lens implants, surgical intervention only occurred for dislocations of the lens; that is, where the lens moved from the original position where placed.

The invention contemplates placement in an eye of prisms, graded density lenses, fiber optic lenses Fresnel lenses, mirrors. Some optics may correc astigmatism. All these variations noted above may be used in the vitreous cavity implants, posterior chamber implants, implants partly or wholly in the site of the natural lens, and anterior chamber implants. Furthermore, mirrors as well as other optics which reflect wholly or in part certain light frequencies may be used. These may allow other frequencies to pass through and/or be absorbed by the mirror or its coatings as required.

The invention also contemplates placement of refracting systems which have gaseous or liquid or vacuum filled chambers on one side or both of a refracting element or elements (e.g. lenses). FIGS. 12A and 12B show side and front views, respectively, of a solid convex lens 20 sandwiched between front and rear chambers 20F and 20R, respectively, which comprise hollow chambers, evacuated or filled with gas or other fluid. This arrangement may increase the amount of refraction (bending of light) possible and affect electromagnetic radiation such as light in other ways. Likewise the reverse arrangement may be used. A solid refracting material may surround a central gaseous or vacuum or fluid filled cavity. FIGS. 13A and 13B are side and front views, respectively, of a hollow convex element 21 sandwiched between solid front and rear elements 21F and 21R, respectively, each having a plane outside surface and concave inside surface. The surfaces (internal or external or both) may be coated or not (e.g. as for anti-reflectance). Entire optical systems may be enclosed (domed) and completely or partly surround another enclosure or enclosures (chambers) each having gases and/or liquids with refractive indexes or other optical properties different from aqueous or vitreous humor. Thus gas, liquid, vacuum and solid combinations, and multiple chambers of each may be used. In a given implant multiple serial arrangements of chambers filled with gases and/or liquids having different densities, refractive indexes and optical properties may be used.

Some chambers may contain vacuums. Chamber walls may have different indexes of refraction and other optical properties and may safely enclose high refractive index and other materials which may be toxic to the eye. Gases and some liquids provide implant buoyancy effects as well.

Intraocular optical combinations alone, or also single or simpler optical devices alone or combined as required with specific eye glasses or contact lenses to produce various effects such as a telescope magnification effect for near and also for distance, and minification of images and the widening of the visual field, and converging (plus) intraocular and eye glass lenses for magnification or reading, may be used. Optics may be used in the anterior and posterior chambers and the vitreous cavity or combinations of these. A vitreous cavity implant may have plus or minus optical power and connect to an existing anterior chamber lens for changing patient focus from distant to near, or near to distant.

Optical devices may be placed which have no refracting and/or reflecting diopter power but whose optical function is to filter out a certain wavelength or wavelengths of electromagnetic radiation. They may have refracting or reflecting power as well. They may reflect some wavelengths (frequencies) but not others. In some, selective filtering effects, selective reflecting effects, selective refracting effects, or combinations of effects may occur. For example, some mirrors may allow some or all wavelengths of light or electromagnetic radiation to pass through or be absorbed while reflecting others. Optical devices may be placed which on stimulation by one or more wavelengths (different frequencies) of electromagnetic radiation such as light, emit light of a particular wavelength, wavelengths, or band of wavelengths in lesser or greater amounts.

Mirrors, and other optics, may be placed anywhere within the eye, and when they are very large, may require multiple supports. They, as other optics, may have parts of them cut out or absent so as to create specific optical effects.

Enclosed or domed (implant) systems may be entirely enclosed or may allow eye fluids to circulate through them. They may have movable parts or not. Non-domed implants enable easy direct access of instruments for implant modifications or adjustments. The dome (enclosure), such as shown in FIGS. 12, 12B, 13A and 13B, may protect the eye against the implant by containing toxic substances of implant parts, acting as a barrier to fluid turbulence and providing a smooth surface for insertion into the eye. The dome may have other functions such as optical ones, and also may keep gases and/or fluids or vacuums inside while keeping aqueous or vitreous out. Implants may be domed, but have various size openings allowing eye substances to move in and out and enable the insertion of instruments to modify implant function.

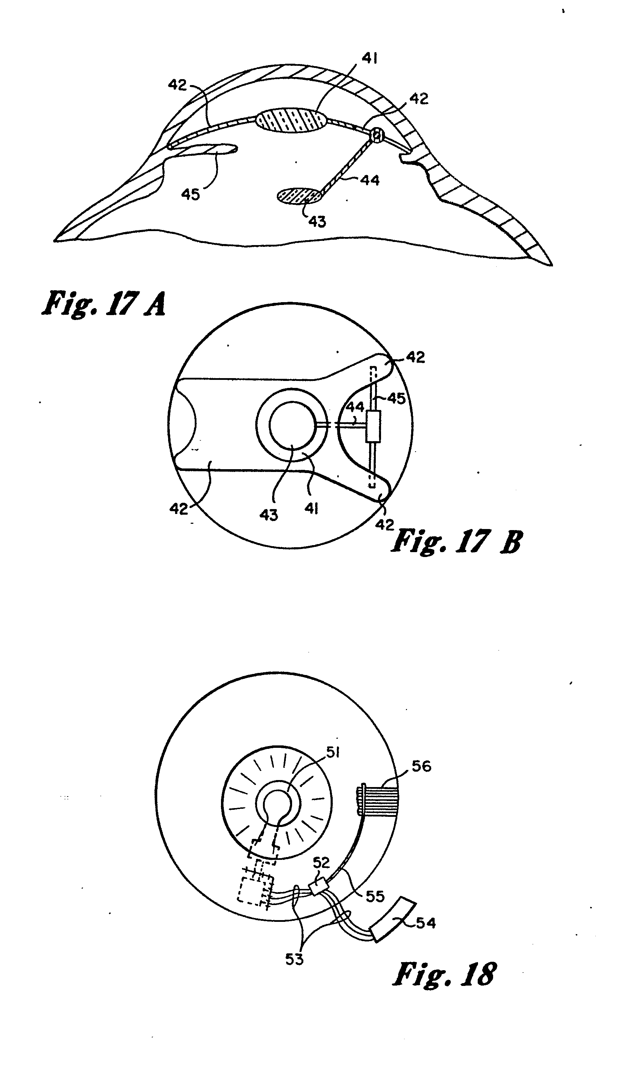

All optical systems may be offset from the eye's geometric center so as to coincide with the true visual axis of the eye. FIG. 14 is a plan view showing a fixed optic 22 centered over the visual axis 23 of the left eye and supported by feet 24 and 25. A movable optic 26, such as a lens, may be supported for movement about a pivot 27 carried by a haptic 33 and may include one or more of a float such as 29 and/or weights such as 28, that may or may not be of a magnetic substance. Optic 26 may then be selectively positioned over or displaced from visual axis 23 as indicated by arrow 31. Selective positioning may be effected by using a magnet to attract weights 28 to the desired position. Alternatively, the wearer may use head movements to position optic 26, and stops such as 32 and a guide such as 2 may be used to help keep element 26 in the desired position.

Numerous combinations of optics may be assembled according to the invention. Mixing of different categories in a given eye may be done (e.g. lens-mirror-filter combinations). One or more of any of these devices may be fixed or movable or deformable-reformable by patient or animal action.

"Multiple powers" in a given optic -- may be patient or surgeon movable, or may be fixed (nonmovable). Each of such optics may have different powers at its different areas. Any such optic may have properties of other optics. For example, a refracting element may be a light filter or have a light occluder as part of it. Bifocal and progressive addition lenses are examples of spectacle multiple power optics.

Anterior chamber implants, posterior chamber implants, and vitreous cavity implants, or implants in the site of the natural lens may have optics, haptics, arms, and other structures held in cradles or holders under tension or have other releasable arrangements. The optics or implant parts can thus be removed or replaced as required.

Nonmovable optics may function in front or between or behind movable optics or vice versa. More than one nonmovable or movable optic or combination of optics may be used in a given eye.

For protection during intraocular implant placement, coatings such as chondroitin sulfate, or soft contact lens, or similar materials may be used.

All optics may be constructed and/or coated or not to filter fully or partly certain light or electromagnetic radiation frequencies or reduce reflectivity of all or some frequencies, or both. This includes enclosures (domes).

Right eye and left eye mirror image configuration implant designs may be made for both anterior chamber and posterior chamber, and for vitreous cavity implants. These may also be made for each right eye and left eye where required. Implants may be made for insertion from all the different hours of the clock (360°) depending on patient need and surgeon's choice. The malleability of the parts used in some anterior chamber, posterior chamber, and vitreous cavity implants enable the surgeon to modify further the configurations and functions.

The invention contemplates different extents of movement for implant parts depending on patient needs and surgeon's choice. FIG. 15 shows a front view of a sliding optic 33 with magnetic substances in the haptic 34 slidable in a guide 35 so that a magnet and/or head movement may be used to selectively position optic 33.

Adjustable stops and guides may also modify left-right and right-left extent of movements and up-down or oblique or anterior-posterior extent and secondary, tertiary, and subsequent movements of implant parts as well. Friction may be provided as required by the contours, guides and hinges and their sleeves, bearings, rods, ratchets, pinions, stops and wherever parts contact each other. Guides may be altered to produce other than a uniplanar movement, such as secondary arcs or curving movements of implant parts (e.g. "S"-shaped and other movements). This can be true for any moving system, such as swinging, sliding, float, and rotational and revolutional systems or combinations of them. All this applies to intraocular and extraocular implants.

Parts may have depressions, hooklets, jogs (bends), holes, protrusions or other variations to enable them to be grasped, engaged, pushed, pulled, or otherwise moved by instruments intra-operatively or postoperatively and so allow the surgeon to modify the function and positions of implants or their parts. FIG. 16 shows a support rod 34' carrying an optic 36 and formed with nonslip ridges 35' to facilitate manipulation by the surgeon to position optic 36 as desired.

The vitreous cavity is that place where vitreous resides in the normal eye. Thus implants which are in the vitreous cavity are posterior to; or posterior and: lateral to; medial to; superior to; or inferior to (or combinations of these as the case may be) the site of the natural lens even if it has been removed. There is not only more room for the optical implant (and other implant systems) in the vitreous cavity, but by posterior placement the peripheral field of vision may remain unobstructed while central optical effects (e.g. magnification of image) can be created. The invention contemplates placement of intraocular pressure controlling implants and drug or chemical delivery implant systems anywhere in and on the globe and also extraocularly.

Vitreous cavity implants are advantageous because the extra space in the vitreous cavity allows for complex as well as simple implants, which may be at and also entered into the eye at considerable distance from iris and cornea and mostly also from other easily damageable internal eye parts. Cornea and iris have traditionally been damaged by intraocular implants.

Implants may be placed in the site of the natural (but removed) lens, the vitreous cavity and the posterior chamber all simultaneously. The vitreous may be normal or not, intentionally removed or not (in standard or usual surgery, vitreous is removed because of the surgical complication of "vitreous loss" or because it is diseased).

Systems are chosen for a particular patient or animal based on his visual requirements. The type of system, its size and parts, its weight, floatation, tinting, magnification or minification, etc., are all determined by the person's (or animal's) anatomy, physiology, age, vocation, avocation, and other factors as the case may be.

Marks, holes and other indicia may be placed on the implants to enable the surgeon to correctly align the optical system and other elements.

Extension parts, opaque lines; markings, diffraction or refracting marks, and other indicia may be made in or near or in alignment with optics, haptics, arms or bars or combinations of them to enable the patient to orient his gaze.

Anterior chamber implants (angle supported implants) may have other optics situated in the posterior chamber or vitreous chamber connected to them by cantilevers or other supports. Anterior chamber implants with widely spaced feet straddling a sector iridectomy may have a hinged and lockable cantilever so as to move a fixed or movable optic into position in the posterior chamber or vitreous cavity. FIGS. 17A and 17B show side and front views, respectively, of an anterior chamber supported vitreous cavity lens. Anterior chamber lens 41 is supported by feet 42 with vitreous cavity lens 43 attached to a hinged-lockable cantilever 44 supported on feet 42. Vitreous cavity lens 43 may also be hinged and moved sideward by one or more of the techniques discussed above and below to a position behind iris 45 when its function is not desired. As stated above in describing materials of optics and support systems, implants herein described may be made of many substances. Anterior chamber implants herein may have feet and/or haptics of silicone rubber; plastic, glass or metal or substances capable of equivalent or similar function and/or combinations of these. Prior art anterior chamber implants have had feet made of Nylon®, polypropylene, or polymethylmethacry-lates. Feet and optic supports according to the invention may be flexible or not, expandable or not.

Posterior chamber implants fixated in the capsular bag or to sclera may support vitreous or anterior cavity optics with cantilevers or other supports. Posterior chamber implants may have any type of supports or feet (e.g. hinged, loop type, solid type feet, etc.) which may be flexible, deformable, elastic or not and made of metal, plastic, glass, silicone rubber, or substances of equivalent or similar function, and may be suturable to the sclera or be epi-sclerally or not. The term "posterior chamber" here means the posterior chamber as anatomically defined (i.e. just posterior to the iris) and/or the site of the natural lens as anatomically described. As with vitreous cavity implants and anterior chamber implants, posterior chamber implants may have movable parts (optics and other elements).

Optical, chemical (drug) delivery and intraocular pressure control implant part movement may occur as follows: by patient or animal head and/or eye movements; by gravity; by moments of force; by inertia (mass); by floats; by magnetic substances and/or electromagnetic radiation transmitters and/or receivers inside and/or outside the eyes -- these magnetic substances and transmitters and receivers may be directly controlled by the patient or not: by electrical properties; by centrifugal and centripetal force; by ultrasound transmitters and/or receivers; by stimulation from mechanical or electrical activity originating in the muscles and nerves of the ciliary body (focus muscles and nerves), and/or by stimulation from mechanical or electrical activity originating in a medial rectus muscle and/or other muscles and nerves in the orbit or periorbital areas. Also, movement may be partly controlled and influenced by friction, stops, arm lengths, and other means. Optic or other movable implant parts may be arranged to move in any and many directions. Implants with moving parts may be intraocular or extraocular.

FIG. 18 is a diagrammatic representation of a front view of an eye showing the relationship between an extraocular and intraocular implant. An intraocular implant 51, which may be focusable having a solenoid and hydraulic system, may be connected to an episcleral implant 52 that may include one or more of a microprocessor or radio receiver coupled by electrical conductors 53 from extraocular (sub or epiperiosteal) rechargeable battery implant 54. Focusing may be triggered by a signal transmitted on link 55 from medial rectus muscle 56.

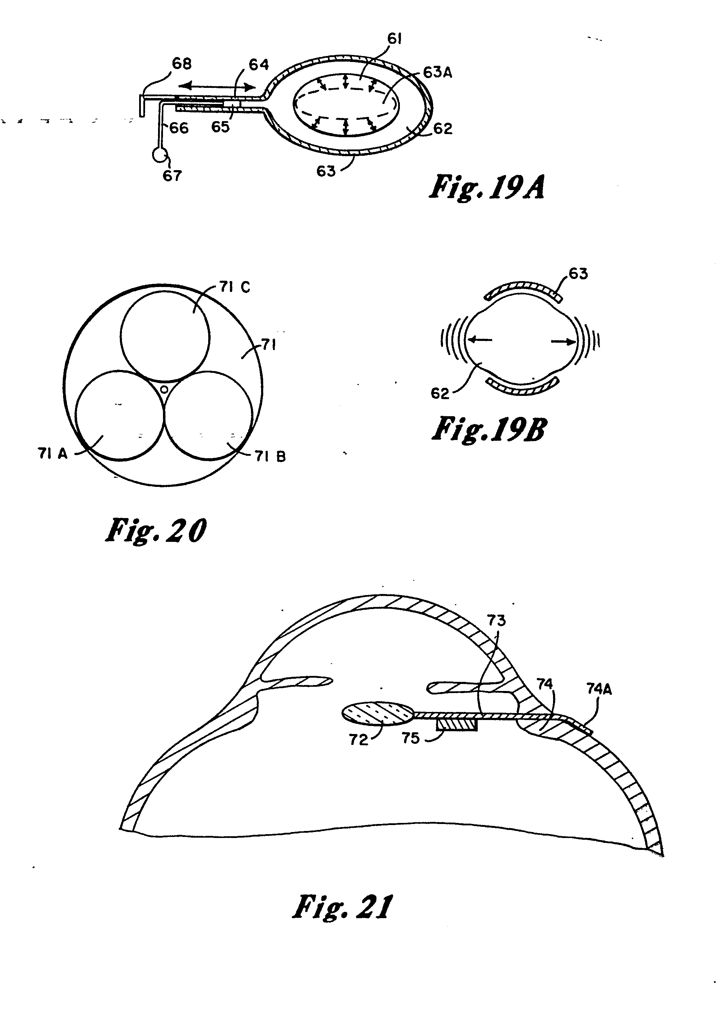

Intraocular and extraocular hydraulic systems may also be used. These may be connected to weights, floats, and other devices, so that head or eye movements activate them or magnetic substances, or other means may activate them. Hydraulic systems may be activated by screw type systems. Pistons which slide or screw into threaded or smooth cylinders and/or vises or clamps or peristaltic type mechanisms which activate hydraulic and optical systems may also be used. Valves may be used with hydraulic and optical systems. Solenoids may activate pistons. Hydraulic systems and other mechanical systems may be used to move optics or deform or change the shape of optics so as to change their powers. To accomplish this, optics may be made with fluid and/or plastic and/or gel interiors and/or substances capable of the same or similar function or combinations of all of these, and they may have deformable, reformable (elastic) envelopes, open or closed appendages of which may be acted on by clamps, vises, pistons, and other elements. They may also be wholly or in part made of a deformable-reformable elastic substance or substances, such as silicone rubber. Envelopes of an optic may have regions of varying strength and/or thickness so as to alter optic expansion and contraction and therefore sphericity, asphericity and other optical properties. FIG. 19A shows a shape changing variable focus optic 61 with a restraining ring 63 around an elastic transparent envelope 62 filled with fluid 63A and connected to a lever 66 that may have a weight or float 67 and restricted in rotational or axial movement by stop 68. Moving piston 65 to the right expands the envelope 62, while moving to the left contracts it thus changing its optical properties. FIG. 19B shows a side view of the optic. Piston 65 may be threaded to mate with threads in cylinder 64 so that rotation of piston 65 by lever 66 effects the desired changes. The system may be fixed in the visual axis or be movable in or out of it. Piston 65 may be a magnetic substance movable on a nonthreaded system.

The mechanical parts of movable implants may include, but are not limited to, those mentioned above in describing support structures, optics and their materials. Parts may be in different and varied arrangements.

Entire movable or nonmovable implant systems may be mounted on or connected to adjustable mounts or connectors so that the entire movable or fixed system can be repositioned relative to the eye and human/animal body. Such mounts may allow self-righting of the moving systems.

Moving and/or fixed implant systems may be placed in front or behind existing implanted optics or the natural lens. Such optics may be simpler, having no main optic, or may be complex, having all the other possible variations, noted, such as among movable optic types.

Telescopes and other complex systems and also simple optics may move in and out of the central visual axis or other useful visual pathways or be fixed in them.

Angles of and extents of and configurations of implant system excursions (sliding, swinging, rotating, floating, and other movement) may be varied depending on the particular eye and the patient (or animal) requirement and the surgeon's preference.

Floats -- may be made of any workable substances, and be of any structure to effect the buoyancy required. They may, for example, be wholly or partly liquid- or gas-filled or have a vacuum within.

Movement may be stopped or altered by bending guides, arms, cantilevers, or special parts or bending or extending special appendages designed for this purpose, or combinations of these. Laser sensitive implant materials may also be used to effect this. by heat contracture or ablation of all of part of these materials. Existing forces cause the movement to new positions after ablation.

An external magnet or magnets or radio frequency transmitters controlled by the patient or another person or being can be used to control the optical, drug, chemical delivery, or intraocular pressure controlling implant systems having a magnetic substance or substances and/or radio frequency receiver or receivers such as shown in FIG.18. With implants such receivers may be activated by impulses, mechanical or electrical, originating with the nerves or muscles of normal eye focusing (accommodation) and/or the medial rectus nerves or muscles or other muscles or nerves serving the eye or periorbital region or through the lid finger pressure on implants with resulting mechanical and/or electrical and other effects. Receivers may activate other systems such as solenoids, hydraulic systems, magnetic systems, and other systems. An external source of controlling energy may be used to release substances from any of the implants for therapeutic or other purposes.

Centrifugal and centripetal force may aide implant movement and/or may be used to keep movable parts from moving in certain head positions during saccades, especially in face down positions via guide configuration and/or hinge friction or other arrangements.

Guides may pass through optics or haptics of the moving optics or through arms or other moving parts of implants so as to secure them in addition to guiding them.

Movable systems may have multiple hinged arms which have the same or different planes of arc. Hinges may have different stops allowing different extents (arcs) of movement. Arm lengths may also differ. Hinges may be combined with extensions, and other elements.

Surface contours of movable systems may be varied in manufacture so as to affect the rate and direction of movement of those systems in the eye. In addition, the position of surface parts may be changed by the surgeon before or after implantation to alter system movements. This may be done by bending or moving parts connected to the implants for that purpose. This affects the drag and/or planing, or other movement of the moving parts. Laser sensitive implant materials may be used to effect this also.

Implants may transmit back (feedback) information to other implants or hand-held (external) receiver-transmitters whose associated receivers may thereby modify their actions (transmissions) so as to allow correct optical implant movements, release chemicals or control of extraocular pressure or release of eye substances.

Magnetic substances may be connected to one or more places of the movable systems: such as mirrors, lenses, filters, prisms, conduits, depots, cantilevers, haptics, swing lens arms, slides, stops, guides, through and through double wall partial or full thickness perforating supports, feet, valves, and other elements. Nonmovable sections or parts may have them as well.

Rotating optical banks (or batteries) may be used to enable patient or doctor to alter optics. FIG. 20 shows a rotatable disk 71. They may be of special use in growing or stretching eyes. The forces and mechanisms used for bank movement may be those described in connection with movable implants and implant parts. Optics, such as prisms or prism systems, may be in such banks (or batteries) or be individually rotatable around an axis so that incoming light rays to the eye are directed to retinal areas with better function as required.

Rotating implants may be ringed, usually at the periphery, with a magnetic substance or substances to aide their movement. Guides, too, may have magnetic substances.

Numerous design inter-relationships among the factors identified in describing movable implants are available within the principles of the invention. Parts, sizes and mechanical arrangements allow even more variations. An implant part has mass and thus has inertial effects -- yet the part may be weightless; that is, buoyed by another part or by buoyancy of its own. Magnetic fields can influence movement by way of affecting magnetic substances of an implant, yet the movement may be also aided by gravity or inertia, and other factors. Floats by their floatation and their mass and contours can also profoundly alter implant movements. They may counter inertial (mass) and other effects, leaving the float or magnetic effect, or neither dominant as the case may be. They can affect frictional, magnetic and gravity effects as well. thus float action may counter, balance, counterbalance, enhance or cancel other effects wholly or in part and may also become the major effect. Some systems may use mostly the effects of mass (inertia) and gravity and head and/or eye movements.

Movable optics, either "plus" or "minus" power, connecting to anterior or posterior chamber or vitreous cavity implant systems for purposes of changing focus of the eye from either distance to near, or near to distance, may be used.

Scleral fixed posterior chamber, vitreous cavity and also anterior chamber implants may require intraoperative or pre-operative or post-operative retinopexy. This may be true for transciliary body and is especially true for trans-retinal entry. FIG. 21 shows a depot implant 75 connected to cantilever 73 secured to sclera 74 with episcleral foot 74A and carrying optic 72.

The invention contemplates episcleral, intrascleral or endoscleral feet of sufficient area, strength and configuration to support and stabilize episcleral (subconjunctival and/or Tenon's capsule) or intra-scleral or intraocular or orbital implants. Such feet may be contoured to conform to the curvature of the globe. Feet may be placed intracorneally and/or at the limbus, and/or sclerally as described herein. In any of these placements, they may support anterior, posterior or vitreous cavity implants by the methods described herein.

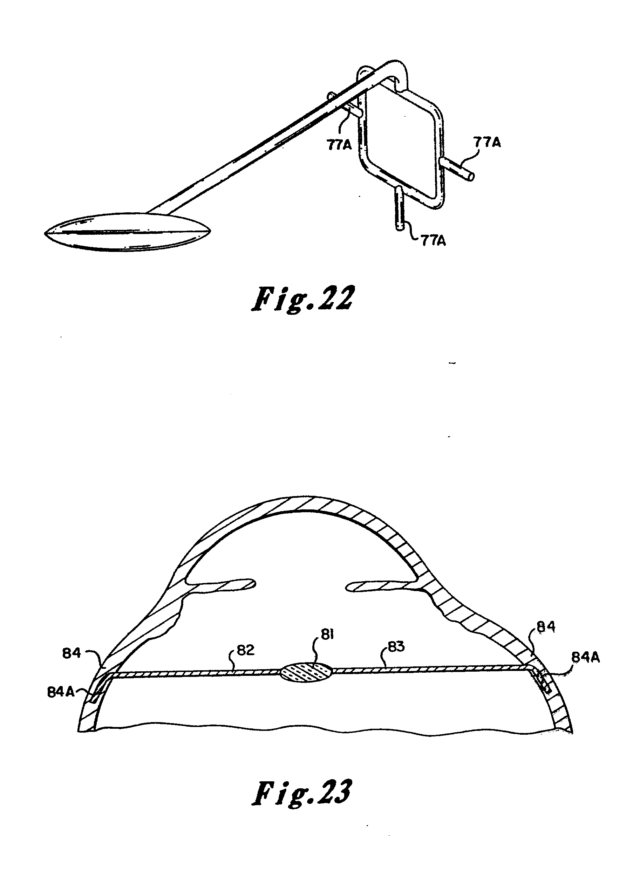

FIG. 22 shows an episcleral foot which has extensions off its periphery to enable better suturing and improve stability. Cantilever supports may be connected to the above feet to support and stabilize episcleral (subconjunctival and/or Tenon's capsule) or intrascleral or intraocular or orbital implants. Cantilevers may be supported by other cantilevers or double wall transverse paraxial or axial (bar, etc.) supports or by posterior chamber or anterior chamber implants.

The invention contemplates double wall transverse supports -- rods, (multiple or single) bars, and other elements, (multiple or single) supports which traverse the posterior (eye) chamber and/or vitreous cavity and having opposite wall episcleral, endoscleral or intrascleral feet of sufficient area to secure them.

FIG. 23 shows a side view of a lens 81 supported in the vitreous cavity by transverse or double eye wall supports 82 and 83 fastened to opposite sides of sclera 84 by intrascleral feet 84A. Transverse supports may be axial or paraxial and situated at different angles. Single or multiple cantilevers or arms connecting to the transverse double wall support structures may help support implant systems (fixed or movable). Multiple separate implants may be used.

The cantilever or double wall support (large) jog or bend is advantageous because it more easily allows the safe placement of an implant with a mostly closed adjacent or nearby intrascleral pocket. The jog or bend allows shifting of implant position after the implant has been entered into the eye so as to enable movement of the foot into an intrascleral partly closed and therefore securing pocket. The jog or bend may be made to extend in different directions depending on the full thickness incision-scleral pocket configuration relationship required. Once the implant is in position, the jog also allows for easier and safer intraocular implant position changes.

There may be five scleral foot fixation-arm/cantilever/transverse support relationships; namely,

(1) with the foot episcleral and the arm or handle also episcleral;

(2) with the foot intrascleral (in a pocket) and the arm or handle episcleral;

(3) with the foot episcleral and the arm or handle intrascleral;

(4) with all parts intrascleral (closed over by sclera by use of a lamellar flap or flaps, or grafts); and

(5) with the entry wound under an episclerally, endosclerally or intrasclerally placed foot with cantilevers and/or transverse double wall supports entering the wound (vitreous cavity) under the foot or in the pocket or both as the case may be. In all cases, conjunctiva and/or Tenon's capsule may be advanced to cover the areas where required.

Feet having a spring type action which tend to expand and press against inside scleral pocket walls may be used. The expansion may also be induced by a bent bar or part which is straightened by the surgeon when the foot has been placed in the pocket. Inflatable or distendable feet may also be used as may other methods.

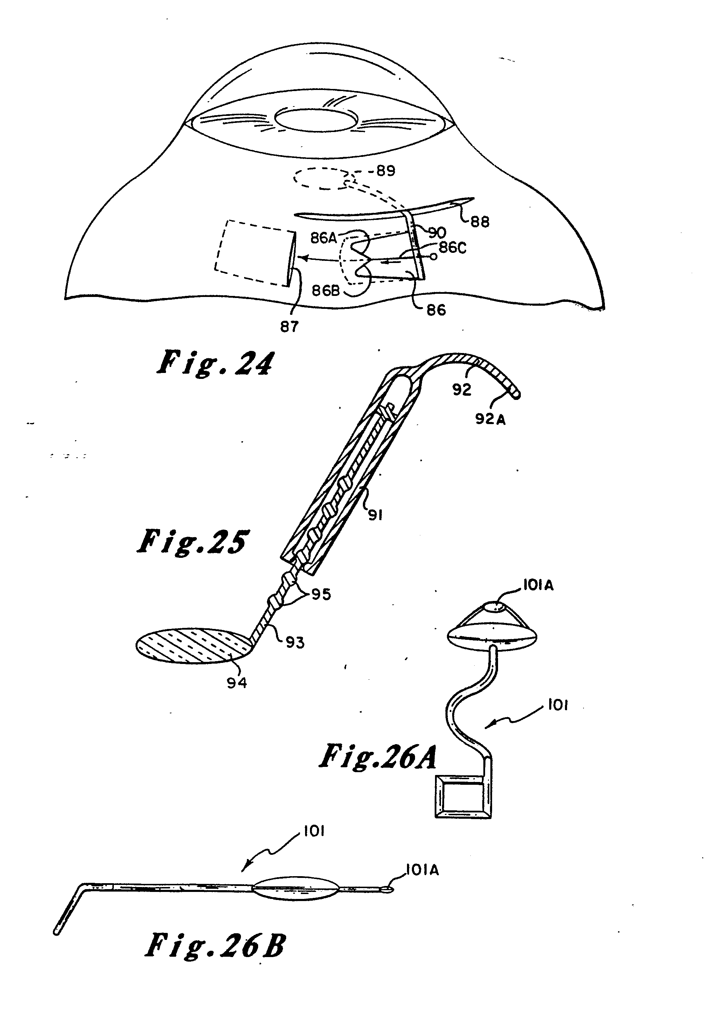

FIG. 24 is a magnified view showing how a foot 86 may be inserted into intrascleral a pocket 87 with optic 89 and support rod or handle 90 passing through a full thickness wound 88 in the sclera into 89 the posterior chamber. Foot 86 may have parts 86A and 86B that enter pocket 87 collapsed and then uncollapse as control member 86C is pressed to seat the foot 86C firmly in pocket 87.

When fixed to sclera, the feet can have varied and sufficiently effective area to stabilize implants, or any other systems or parts requiring support and stability about the eye. The amount of feet, their placements, their sizes, shapes and configurations may be varied to accommodate differing sizes, configurations, and (mass) weights of systems or parts, and the foot size, configuration and thickness may be varied to produce scleral buckling effects so as to prevent retinal detachments or treat vitreous traction or retinal breaks or other retinal and vitreous pathology. The feet may be episcleral or intrascleral or endoscleral or combinations of these. They may have notches, grooves, irregularities or openings or combinations in the center or on edges to allow tissue (scleral or otherwise) to grow across, and/or be sutured to sclera, and/or they can be made of substances capable of cellular invasion by human cells and tissue, all of this improving their fixation. They can be fixed to sclera anywhere except where optic nerves enter or where sclera is too thin. The invention contemplates foot fixation at, adjacent to, or nearby the entry incision of an intraocular implant, as well as at a greater distance from it, as for example, on the opposite side of the globe. Feet with handles, cantilevers or rods (etc.) having jogs, notches or other variations along their lengths may be secondarily placed to modify or stabilize an implant system.

Scleral pockets (for intrascleral feet) not only enable a sealing of the eye using the eye's own natural wall, but also greatly increase the operative safety of intraocular implant insertion. Pockets provide future safety by firm fixation of implant systems. Intraoperatively a pocket may be partly closed or be fully open, having one or more lamellar scleral flaps to be closed over a foot. Flaps may be partly sutured before foot placement as well. A pocket may be open on one side only, thereby giving extra security to a foot and requiring less suturing (gluing). Pockets may be anywhere except where the optic nerve enters the eye or where the sclera is too thin. Pockets may be of many

**0162573**

sizes and shapes. Thick or thin sclera may be reinforced by human and/or animal tissue to improve or enable foot fixation.

Entry full thickness incisions may be wholly or in part, at the limbus to anywhere on the sclera except where the optic nerve enters. The limbus is the area of the junction of sclera and cornea. Incision sites are varied depending on the patient's individual requirements. Transretinal implants enter the eye more posteriorly, where the eye muscles are further apart. This gives space for large feet and enables more foot-entry incision relationship possibilities without having to retract muscles. Full thickness incisions may be varied. For example, they may be stepped, slanting, or perpendicular to the coats of the eye. They may be any length and any pattern ("S"-type, straight, "Z"-type, "L"-type, "U"-type, curved, or other shapes or combinations). They may be within an intrascleral pocket, under the implant foot, in an episcleral foot fixation, or adjacent to the pocket or episcleral foot or distant from it, etc. They may share a common episcleral tissue (lamellar) flap or may have separate scleral flaps or none at all, depending on the individual eye's or surgeon's requirements. They may be closed with sutures, glue or any other suitable methods.

Many implant parts may be malleable to enable the surgeon to alter the implant position or foot, contours as needed. In addition, folded ribbon or coil or corrugated arrangements may be placed in cantilevers, arms and double (eye) wall supports to enable adjusting the position of implants or their parts by the surgeon. Adjustable rod and sleeve type and other extensions may also be used for this purpose.

Extensions (such as rod and sleeve type) may be used to (1) enable safer placement of implants so as to extend them within the eye only after they are securely fixed to the sclera)(globe); (2) to enable the patient to move a system in and out of use by head movements, magnetic substances, or other techniques and (3) to enable "play" or automatic adjustability of length in a double (eye) wall (transverse) support so as not to put unnatural stresses on the eye wall and foot fixation when the globe is compressed, pulled or pushed upon. Spring and/or elastic arrangements may also be used alone or in combination with the rod-sleeve type extensions for this purpose.

FIG. 25 shows an extensible support comprising a sleeve 91 supported from a malleable portion 92 and foot 92A and carrying an extensible rod 93 supporting optic 94. Rod 93 may carry a number of axially spaced elevations, such as 95, for engaging the lip of sleeve 91 and allowing the surgeon to position extensible arm 93 at a desired position.

Preoperative marking of the eye to indicate its vertical and horizontal meridians, or other features, is often necessary as well as preoperative measurement of pupil size and position. Furthermore, saccade and other eye movement studies as well as the range of motion of the patient's neck (and spine) may be needed. These studies are necessary when placing some movable implant systems. Preoperative ultrasound measurements of scleral thickness and infrared blood vessel imaging may be required.

Simultaneous filtering sclerectomy or sclerotomy may be created as needed to lower the intraocular pressure and allow the implant entry wound and the scleral pocket or episcleral site to correctly heal.

The following is one of many possible surgical approaches to vitreous cavity implants and posterior chamber implants which are intrascleral foot fixed. Preoperative studies and marking are recommended for most movable implants. Such studies may be of use in nonmovable systems as well. FIGS. 26A and 26B are perspective and edge views, respectively, of an assembly 101 with a nonmoveable float 101A, an optic, jog and foot for implanting according to the invention.

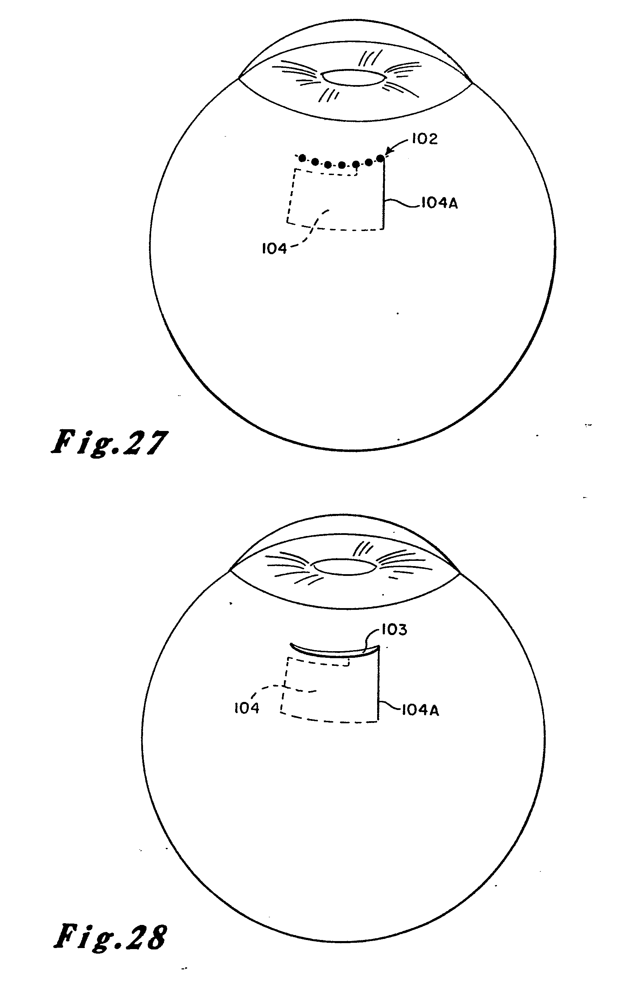

The conjunctiva and Tenon's capsule are incised and tracted away. Cautery is applied as required. The measurements of the distance from the limbus (corneal-scleral junction) to the intended full thickness eye incision (for implant entry) is made using calipers. The relationships of the intended incision site to the vertical and horizontal (premarked ocular (globe) meridians is noted and may be measured with a small sterile protractor. For each scleral foot supported implant style there are recommended distances between globe entry incisions, scleral foot fixation placements, and globe meridians. If a pocket is to be made, it is placed accordingly. Incision-pocket-globe meridian orientation and marking devices may be provided for the surgeon's use. The correct intended dried incision site 102 is marked with a thin-tipped sterile dye marker as shown in FIG. 27. An intrascleral pocket 104 with one end open 104A may be created with a knife and/or blunt or semi-sharp instruments adjacent to the intended full thickness implant (intraocular) incision 102 as shown in FIG. 27. The correctness of the pocket size is tested with a "test foot" instrument of the same size and shape as the foot (feet) of the implant to be used. The human lens may have been previously removed. If not, it may be then removed by any method desired, and the implant incision is made by entering at the premarked (incision) site 103 FIG. 28. Vitreous may be removed or not. Healon® (sodium hyaluronate), and/or other substances, may be used to move vitreous away or, if the vitreous is removed, air, Healon®, or substances of the same or similar function may replace it during surgery. There may be liquid, air or other inflow, into the globe from the same or another entry site or sites.

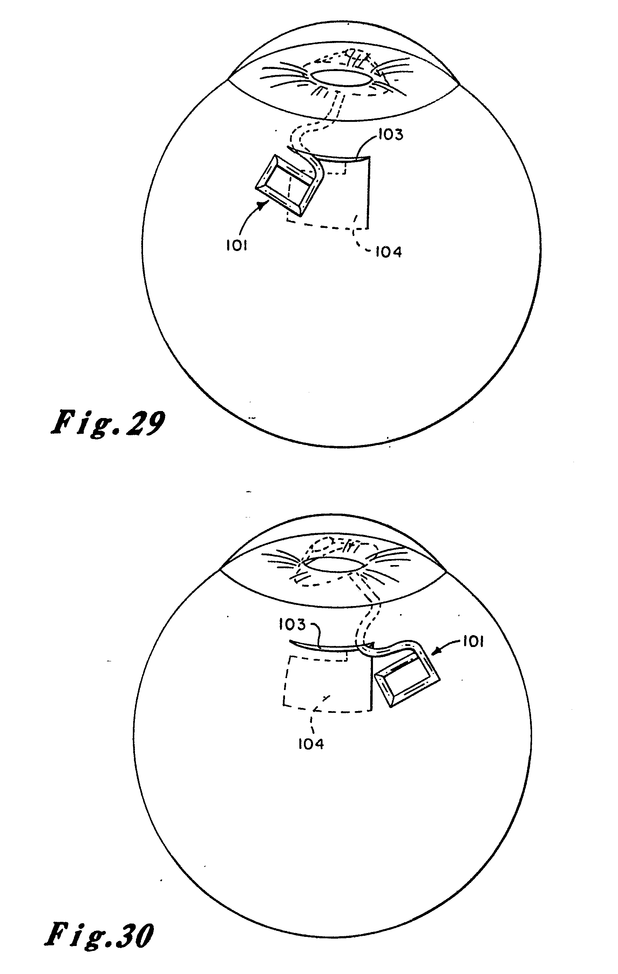

The implant 101 is placed through the full thickness incision 103 into the eye and its foot is placed into the scleral pocket 104 as shown in FIGS. 29 and 30. It is here that the value of the large jog or bend in the cantilever (or jog bend such as this in any intraocular support) becomes apparent because it more easily and safely enables a secure foot into scleral pocket placement at a potentially dangerous time. Depending on the implant required (and therefore the scleral pocket-entry incision relationship) the jog may be made in many directions. In summary when the implant enters the eye, the large jog or bend enables the surgeon to more safely shift the foot (and implant) to a position where the foot can be placed into an open or partly open scleral pocket.

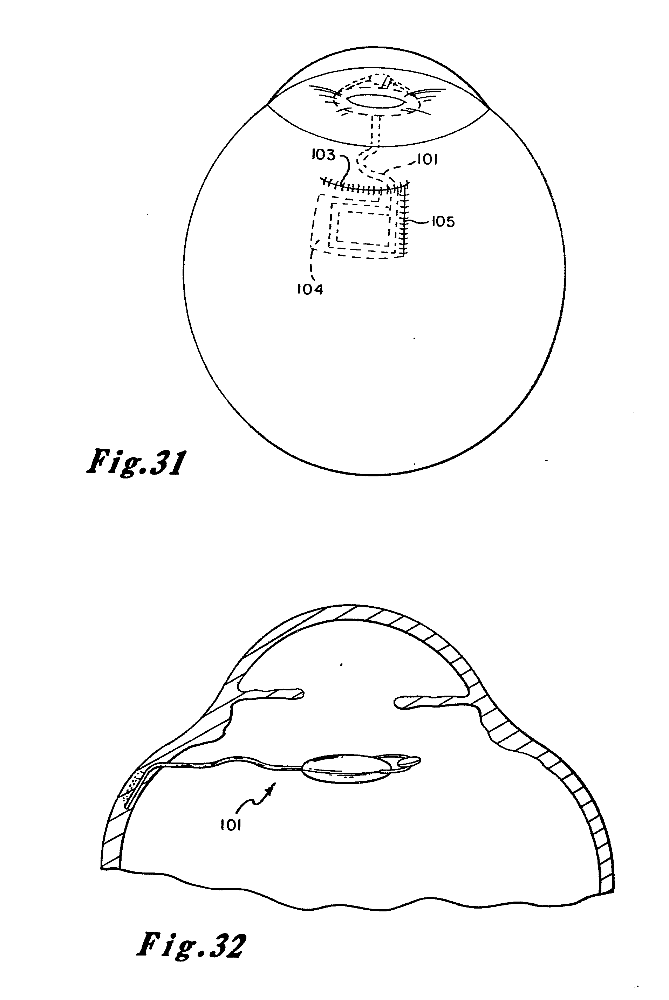

Some implants may have a "handle" where they can be safely and easily grasped with existing instruments or those designed specifically for the purpose. The entry wound is usually closed with permanent sutures 105 as shown in FIG. 31. The entry lamellar scleral flap which was raised to create the pocket was originally extended to the entry incision. It is also closed with permanent type sutures. The overlying conjunctiva and Tenon's capsule which were incised and moved out of the way are sutured back to their original positions. The globe intraocular tension tenses the sclera and now enables (intraoperative) adjustment of the implant if needed, using small intraocular instruments while viewing the implant through the pupil (FIG. 32).

Glycerin may be used to dehydrate the sclera so that the surgeon can see the depth of the knife or instrument while making the pocket, but some surgeons may prefer to thicken the sclera with sterile water or hypotonic solutions instead.

Drug or Chemical Delivery Implant Systems may have roles in the therapy of uveitis, endophthalmitis, neoplasms, infectious diseases, metabolic, degenerative, genetic and other disorders (e.g. diabetes, retinitis pigmentosa, and other disorders). The methods may include:

1. Conduit or channel methods (a hollow or grooved implant part or parts which allow drug flow into and/or out of the eye) and separate infusion and aspiration passages may be provided;

2. Conduit or channel methods combined with valves and/or differentially permeable parts or membranes;

3. Depot methods with the replaceable (or not) depot or depots of drugs or chemicals connected to implant support structures or any parts of implants;

4. Perforating, self-sealing methods -- needles for injection of substances penetrate self-sealing membranes or barriers; and

5. Combinations of the above four.

Implants may also be a source of electrical energy in or on or near the eye so as to facilitate or limit drug penetration and/or produce other biologic effects. Drug or chemical delivering extraocular implants may function alone, without associated intraocular drug or chemical delivery, or other implants.

Barriers or membranes which are differentially permeable, may be used with both depot and conduit systems. Drug or chemical delivery depots may be within the eye, attached to or within implant parts, or may be connected to episcleral, intrascleral, orbital or periorbital implants.

Depots are implant parts which may be permanently connected to implants or may be removable. They contain drugs or chemicals, the release of which may be controlled by valves or other means. Such depots may be resuppliable with drugs or chemicals. They may be combined with optical or intraocular pressure controlling implants or both.

Barriers or membranes selectively permeable to some chemicals but not to most or all bacteria or viruses may be used with both depot and conduit systems. FIG. 21 shows a side view of a posterior chamber lens 72 supported from a cantilever 73 supported by the sclera 74 using episcleral foot 74A with cantilever 73 carrying a depot 75. Depots may be within the eye, attached to or inside implant parts, or may be episcleral, intrascleral, orbital, or periorbital.

The implants may alter intraocular pressure. Outflow of intraocular fluids may be controlled by conduits or channels of various sizes which may have adjustable valves or other impediments to control the outflow rate of aqueous humor. Such implants may be used as collectors of intraocular substances, as may some drug and chemical delivery implants.

Photo cells, photovoltaic cells, or any devices that convert electromagnetic energy to galvanic or other current, rechargeable or nonrechargeable batteries for producing and/or storing electricity, electromagnetic radiation detection devices, electronic circuitry, e.g. radio circuits and circuits enabling pre-programming and/or programming and/or controlling of implant activity or combinations of these may be implanted intra- or extraocularly or both. Programming may also be used in external radios or ultrasound transmitters and receivers which may be used in combination with the implants.

Using any or all of these methods the devices may be fixated by connection to intraocular sclerally and/or limbally and/or corneally fixed implant parts as well as extraocularly orbitally and periorbitally fixed implant parts or combinations of all these so as to provide therapy introcularly, orbitally, periorbitally or a combination of these. Orbital implants may be fixed episclerally as well.

There has been described novel apparatus and techniques for improving vision with eye implants. It is

31

0162573

evident that those skilled in the art may now make numerous uses and modifications of and departures from the specific embodiments described herein without departing from the inventive concepts. Consequently, the invention is to be construed as embracing each and every novel feature and novel combination of features present in or possessed by the apparatus and techniques herein disclosed and limited solely by the spirit and scope of the appended claims.

What is claimed is:

Any support, such as a cantilever or transglobal support, may carry multiple implant's, movable and/or stationary, such as optics.

## CLAIMS

1.    Apparatus for vision maintenance or improvement of a living being comprising, vision treating means (11,15) for affecting the vision capabilities of the living being, the vision treating means comprising a solid, gas, liquid or vacuum optic (11,15); and a haptic (13,14) for supporting the optic in the eye of the living being so as to intercept the visual axis thereof.

2.    Apparatus for vision maintenance or improvement of a living being comprising, vision treating means for affecting the vision capabilities of the living being, the vision treating means comprising a deformable elastic optic (61); and a haptic for intraocularly supporting the optic inside the eye of the living being.

3.    Apparatus for vision maintenance or improvement of a living being comprising, vision treating means for affecting the vision capabilities of the living being,the vision treating means including a mirror optic; and haptic means for intraocularly supporting the mirror optic.

4.    Apparatus for vision maintenance or improvement of a living being comprising, vision treating means for affecting the vision capabilities of the living being, the vision treating means comprising at least one optic (26); and a haptic means (33) for intraocularly supporting the optic, the optic comprising an element (26) in an optical system along the visual axis of the eye of the living being having a magnification different from that of the natural eye.

5. Apparatus for vision maintenance or improvement of a living being comprising, vision treating means for affecting the vision capabilities of the living being, the vision treating means comprising an optic (43); haptic means for intraocularly supporting the optic (42); and means (44,45) for selectively positioning the optic, while intraocularly implanted, to selectively change the effect of the optic on the vision capabilities of the living being.

6. Apparatus for vision maintenance or improvement of a living being comprising, vision treating means for affecting the vision capabilities of the living being, the vision treating means inclusing an implant within a dome enclosure intraocularly supported in the eye of the living being.

7. Apparatus for vision maintenance or improvement of a living being having an eye characterized by a visual axis, the apparatus comprising, vision treating means for affecting the vision capabilities of the living being, the vision treating means including an optic (26) and means (13) for intraoculalrly supporting the optic with the optical axis coincident with the visual axis.

8. Apparatus for vision maintenance or improvement of a human being comprising, a plurality of optical elements (11,15') inside the eye; and means (13',14') for supporting the elements in spaced relationship along the optical axis of the eye.

9. Apparatus for vision maintenance or improvement of a human being comprising, an optical element in the eye; and means including a transglobe bar for supporting the optical elements in said eye.

10. Apparatus for maintaining or improving vision in human being comprising, an element inside the eye for improving or maintaining vision; and transretinal support means for supporting the element within the eye.

11. Apparatus for maintaining or improving vision in human beings comprising, an element (11) inside the eye for improving or maintaining vision; and support means (13) extending from at least one foot (77) secured to the sclera of the eye for supporting the element, the foot having multidirectional stability.

12. Apparatus for vision maintenance of improvement of a living being comprising, vision treating means (72) for affecting the vision capabilities of the living being, support means (73,74A) fastened to the outside wall of an eye of the living being for supporting the vision treating means inside the vitreous cavity or posterior chamber or anterior chamber of the eye.

13. Apparatus for vision maintenance or improvement of a living being comprising, vision treating means (89) for affecting the vision capabilities of the living being; and means (90) for implanting the vision treating means sufficiently close to the eye of the living being to affect the vision capabilities of the eye, the vision treating means comprising medication.

14. Apparatus in accordance with any of claims 1 to 13, wherein the vision treating means further comprises at least one intraocular implant (89).

15. Apparatus in accordance with any of claims 1 to 14, wherein the vision means comprises at least one

extraocular implant.

16. Apparatus in accordance with claims 14 and 15, further comprising, means for intercoupling the at least one intraocular implant and the one extraocular implant in cooperative relationship so that the extraocular implant aids the vision treating action of the intraocular implant.

17. Apparatus in accordance with claim 14, wherein the intraocular implant is an optic.

18. Apparatus in accordance with claim 17, wherein the optic comprises three contiguous portions, at least one of which is solid refracting material and at least another of which is a cavity.

19. Apparatus in accordance with claim 18, wherein the cavity is evacuated.

20. Apparatus in accordance with claim 18, wherein the cavity is fluid-filled.

21. Apparatus in accordance with claim 5, wherein the means for selectively positioning includes means for selectively positioning the optic between at least a first position intercepting the optical axis of the eye of the living being and a second position clear of the optical axis.

22. Apparatus in accordance with claim 5 or claim 21, wherein the means for selectively positioning comprises a pantagraph.

23. An eye implant (115) supported in the vitreous cavity of an eye.

24. An implant in accordance with claim 23, further comprising a support rod (13) connected to an element (14) in the eye for supporting the eye implant, the eye including an iris sclera and characterized by a visual axis.

25. An implant in accordance with claim 24, wherein the element is an anterior chamber lens (15).

26. An implant in accordance with claim 24, wherein the element is a support (14) for an anterior chamber lens.

27. An implant in accordance with any of claims 23 to 26, wherein the support rod is curved (13") away from the visual axis.

28. An implant in accordance with any of claims 23 to 27, wherein the support rod is connected to the sclera.

29. An implant in accordance with claim 24, wherein the element is a posterior chamber lens (15').

30. An implant in accordance with claim 29, further comprising at least one other support rod connected between the eye implant and the posterior chamber lens.

31. An implant in accordance with claim 24, further comprising at least one other of said eye implant and or said support rod.

*Fig. 1*

*Fig. 2*

*Fig. 3*

*Fig. 4*

*Fig. 5*

*Fig. 6*

0162573

*Fig. 7*

*Fig. 8*

Fig. 9

Fig. 14

*Fig. 10*

*Fig. 15*

*Fig. 16*

*Fig. 11*

*Fig. 12 A*

*Fig. 12 B*

*Fig. 13 A*

*Fig. 13 B*

**Fig. 17 A**

**Fig. 17 B**

**Fig. 18**

*Fig. 19A*

*Fig. 20*

*Fig. 19B*

*Fig. 21*

77A

77A

77A

*Fig.22*

84

84

84A

82 81 83

84A

*Fig.23*

Fig. 24

Fig. 25

Fig. 26A

Fig. 26B

Fig.27

Fig.28

*Fig.29*

*Fig.30*

*Fig.31*

*Fig.32*